# EUROPÄISCHE PATENTSCHRIFT

(11) **EP 1 487 364 B1**
(45) Veröffentlichungstag und Bekanntmachung des Hinweises auf die Patenterteilung: **09.05.2007**
(21) Anmeldenummer: 03744713.3
(22) Anmeldetag: 21.03.2003
(51) Int. Cl.: A61B 18/00

(54) **MEDIZINISCHES INSTRUMENT ZUR BEHANDLUNG VON GEWEBE MITTELS HOCHFREQUENZSTROM SOWIE MEDIZINISCHES SYSTEM MIT EINEM DERARTIGEN MEDIZINISCHEN INSTRUMENT**
MEDICAL INSTRUMENT FOR TREATING TISSUE BY MEANS OF HIGH-FREQUENCY CURRENT AND MEDICAL SYSTEM COMPRISING SUCH A MEDICAL INSTRUMENT
INSTRUMENT MEDICAL POUR TRAITER DES TISSUS AU MOYEN DE COURANT HAUTE FREQUENCE ET SYSTEME MEDICAL COMPORTANT UN INSTRUMENT MEDICAL DE CE TYPE

(30) Priorität: 22.03.2002 DE 10212841
(43) Veröffentlichungstag der Anmeldung: 22.12.2004
(73) Patentinhaber: Karl Storz GmbH & Co. KG, 78532 Tuttlingen (DE)
(72) Erfinder: IRION, Klaus, M., 78576 Liptingen (DE); HAGELAUER, Ulrich, 78464 Konstanz (DE)
(74) Vertreter: Heuckeroth, Volker
(86) Internationale Anmeldenummer: PCT/EP2003/002956
(87) Internationale Veröffentlichungsnummer: WO 2003/079915

(56) Entgegenhaltungen:
- US-A- 5 507 743
- US-B1- 6 190 382
- US-B1- 6 322 559
- US-B1- 6 346 105

## Beschreibung

Die Erfindung betrifft ein medizinisches Instrument zur Behandlung von Gewebe mittels Hochfrequenzstrom, mit zumindest einer mit Hochfrequenzstrom beaufschlagbaren Elektrode, die an einem distalen Ende eines langerstreckten Elektrodenträgers angeordnet ist.

Die Erfindung betrifft ferner ein medizinisches System mit einem derartigen medizinischen Instrument.

Ein Instrument der eingangs genannten Art ist aus der WO 00/53079 bekannt.

Ein Instrument der eingangs genannten Art wird in der Hochfrequenzchirurgie verwendet. Die Hochfrequenzchirurgie wird therapeutisch in verschiedenen medizinischen Fachdisziplinen wie bspw. der Urologie, Gynäkologie, Neurochirurgie, Abdominalchirurgie usw. eingesetzt. Speziell in der Urologie und der Gynäkologie werden mittels eines eingangs genannten Instruments, das auch als Resektoskop bezeichnet wird, endoskopisch Prostatagewebe oder Endometriumgewebe abgetragen. Die endoskopische HF-gestützte Prostataresektion bei gutartig'vergrößerter Prostata ist eine der häufigsten endoskopischen Therapien. Mittels der mit Hochfrequenzstrom beaufschlagbaren Elektrode, die monopolar oder bipolar ausgeführt sein kann, wobei im letzteren Fall zwei als Gegenpole ausgebildete Elektroden vorgesehen sind, wird Gewebe unter der thermischen Wirkung des Hochfrequenzstroms abgetragen und/oder koaguliert und/oder vaporisiert. Die zumindest eine Elektrode ist bspw. in der Form einer Schlinge ausgebildet und wird daher oft auch als Schlinge bzw. HF-Schlinge bezeichnet.

Derzeit werden HF-chirurgische Eingriffe mittels eines Instruments der eingangs genannten Art, bspw. bei der Prostataresektion oder Resektion von Tumoren in der dünnwandigen Harnblase, endoskopisch visuell kontrolliert. Zu diesem Zweck ist das Instrument herkömmlicherweise mit einer Endoskopoptik gekoppelt. Die visuelle Kontrolle des therapeutischen Eingriffs ist jedoch unzureichend. Insbesondere die Einstellung der Schneidtiefe der Elektrode kann endoskopisch visuell nicht beobachtet bzw. abgebildet werden. Wird jedoch zu tief geschnitten, kommt es zu Verletzungen unbeteiligten Gewebes, bspw. der Prostatakapsel im Falle der Prostataresektion. Eine Kontrolle der Eindringtiefe der Elektrode im Gewebe könnte prinzipiell über den Einsatz bildgebender und speziell schnittbildgebender Verfahren wie Ultraschall, Röntgen, Computertomographie oder Magnetresonanz ermöglicht werden. Derartige Navigations- und Lokalisationssysteme sind für die Chirurgie bzw. Endoskopie bereits bekannt. Grundvoraussetzung für die Navigation über derartige Ultraschall-, Computertomographie-, Magnetresonanz- oder andere Bildinformationen ist jedoch die Positions- bzw. Lagebestimmung des Instruments gegenüber einer räumlichen Referenz in der Weise, daß bspw. die Position des Instruments lagerichtig in das Bild eingekoppelt werden kann.

Für die Erfassung einer Position und/oder Lage eines Instruments im Körper scheiden jedoch die Positionsbestimmung über Ultraschall oder Infrarot aus, da für derartige Systeme kein Sichtkontakt zum zu bestimmenden Ort des Instruments im Körper besteht. Eine andere Möglichkeit besteht in einer direkten mechanischen Kopplung des Instruments über Positionsencoder nach extrakorporal, was jedoch aufgrund des dazu erforderlichen großen Bauraums von extrakorporal nach intrakorporal sehr nachteilig ist.

Ein weiteres medizinisches Instrument zur Behandlung von Gewebe mittels Hochfrequenzstrom der eingangs genannten Art ist aus dem Dokument US 6,190,382 B1 bekannt. Bei diesem bekannten Instrument, das Teil eines Hochfrequenz-Katheter-Systems zum Abtragen von biologischem Gewebe eines Körpergefäßes ist, ist die Elektrode am distalen Ende des Elektrodenträgers als entfaltbare Antenne ausgebildet, die Hochfrequenzenergie empfängt und an das Gewebe abgibt. Das Instrument weist Röntgenmarker zur Lokalisierung der Spitze der Elektrode innerhalb eines Körpergefäßes auf.

Aus dem Dokument US 5,273,025 ist eine Vorrichtung bekannt, mit der die Position und Lage eines in den Körper eingesetzten Abschnitts eines flexiblen Schaftes eines Endoskops elektromagnetisch bestimmt werden kann. Zu diesem Zweck sind in dem flexiblen Schaft des Endoskops eine oder mehrere Spulen angeordnet, die von einem extrakorporal erzeugten elektromagnetischen Feld, das in den Körper durch die Körperoberfläche eingekoppelt wird, angeregt werden. Die in den Spulen durch das äußere elektromagnetische Feld induzierte Spannung, deren Stärke von der Orientierung der Spulen zu dem elektromagnetischen Feld abhängt, wird über eine Leitung durch den Schaft des Endoskops extrakorporal zu einer Auswerteeinrichtung geführt und zur Positions- und Lagebestimmung ausgewertet.

Ein weiteres System zur elektromagnetischen Bestimmung der räumlichen Position und/oder Orientierung eines oder mehrerer Objekte ist aus der EP 1 096 268 A2 bekannt. Dieses bekannte System verwendet eine Spule, die von einem extrakorporal erzeugten elektromagnetischen Feld angeregt wird, und die an dem Objekt, dessen Lage und/oder Position zu bestimmen ist, befestigt ist. Das positions- bzw. lagezubestimmende Objekt ist dort ein flexibles Element, das bspw. in einen Katheter eingesetzt werden kann.

Der vorliegenden Erfindung liegt die Aufgabe zugrunde, ein medizinisches Instrument zur Behandlung von Gewebe mittels Hochfrequenzstrom dahingehend weiterzubilden, daß die Eindringtiefe der Elektrode in das zu behandelnde Gewebe möglichst genau kontrolliert werden kann, um Verletzungen unbeteiligten Gewebes durch die Elektrode zu vermeiden.

Erfindungsgemäß wird diese Aufgabe hinsichtlich des eingangs genannten Instruments dadurch gelöst, daß im distalen Bereich des Elektrodenträgers zumindest ein elektromagnetisch wirkender Positionssensor, der in der Lage ist, in einem Magnetfeld ein elektrisches Signal zu erzeugen, der zumindest einen Elektrode unmittelbar benachbart angeordnet ist, derart, daß die intrakorporale Position und/oder die Lage der zumindest einen Elektrode erfaßbar ist.

Durch die erfindungsgemäße Ausgestaltung des Elektrodenträgers mit zumindest einem elektromagnetisch wirkenden Positionssensor, der der zumindest einen Elektrode unmittelbar benachbart angeordnet ist, läßt sich über die Positions- und/oder Lageerfassung die Eindringtiefe der zumindest einen Elektrode in dem zu behandelnden Gewebe genau bestimmen und ggf. in Verbindung mit einem bildgebenden System wie bspw. einem Ultraschallsystem oder einem Magnetresonanzsystem in Relation zu einer räumlichen Referenz bildlich dargestellt werden. Die Anordnung des zumindest einen Positionssensors in unmittelbarer Nachbarschaft der Elektrode hat den Vorteil, daß die relative Lage zwischen der Elektrode und dem Positionssensor während der Manipulation des Instruments durch den Arzt unveränderlich ist, wodurch eine besonders genaue Positions- und/oder Lagebestimmung der Elektrode und damit der Eindringtiefe in das zu behandelnde Gewebe kontrolliert werden kann. Dies ist insbesondere deswegen erforderlich, weil der Elektrodenträger eines derartigen Instruments gewöhnlicherweise aus einem länglichen Gestänge gebildet ist, das sich beim Aufdrücken der Elektrode auf Gewebe elastisch durchbiegt.

In einer bevorzugten Ausgestaltung ist der zumindest eine Positionssensor im Körper des Elektrodenträger nahe der Elektrode eingebettet.

Hierbei ist von Vorteil, daß der Positionssensor nicht vom Elektrodenträger absteht und beim Gebrauch des Instruments zum Behandeln von Gewebe ein mechanisches Hindernis darstellt oder selbst beschädigt wird. Auch die Reinigbarkeit des Instruments wird auf diese Weise erleichtert bzw. im Vergleich zu einem Instrument ohne Positionsbestimmung nicht erschwert.

In einer besonders bevorzugten Ausgestaltung ist der Positionssensor eine Spule.

Die Verwendung einer Spule, insbesondere miniaturisierten Spule, die gemäß der zuvor erwähnten Ausgestaltung in den Körper des Elektrodenträgers nahe der Elektrode eingebettet sein kann, hat den Vorteil, daß das Instrument mit dem modifizierten Elektrodenträger im Vergleich zu einem herkömmlichen Elektrodenträger ohne signifikanten Kostenmehraufwand hergestellt werden kann. Die Verwendung einer Spule hat den Vorteil, daß mit einer Spule bereits fünf Freiheitsgrade der Position und Lage der Spule und damit der Elektrode erfaßt werden können, nämlich die Position der Spule in einem kartesischen Raum-Koordinatensystem sowie zusätzlich noch die Drehlage der Spule um zwei zueinander senkrechte und zur Spulenachse senkrechte Achsen.

Dabei ist es weiterhin bevorzugt, wenn die Spule mit ihrer Spulenachse im wesentlichen parallel zur Längsrichtung des Elektrodenträgers angeordnet ist.

Diese Maßnahme hat zum einen den Vorteil, daß sich die Spule in dieser Lage in den länglichen Körper des Elektrodenträgers nahe der Elektrode einbetten läßt, und zum anderen, daß auf diese Weise die räumliche Lage der Spule relativ zur Elektrode in einfacher Relation wohlbestimmt ist. Der einzige Freiheitsgrad, der mit dieser Positionierung der Spule nicht erfaßt werden kann, ist eine Drehung des Instruments bzw. Elektrodenträgers um die Spulenachse. Ein weiterer Vorteil dieses Einbaus des Positionssensors besteht darin, daß sich die Außenkontur des sensortragenden Elektrodenträgers zumindest im distalen Bereich gegenüber dem Standardelektrodenträger nicht ändert, wodurch Elektrodenträger und Elektrode zu bestehenden Resektoskopen mit Endoskop kompatibel sind.

In einer weiteren bevorzugten Ausgestaltung ist der zumindest eine Positionssensor außerhalb der Längsmittelache des Elektrodenträgers angeordnet.

Hierbei ist es von Vorteil, bspw. wenn wie zuvor beschrieben der Positionssensor eine Spule ist, die in Längsrichtung des Elektrodenträgers angeordnet ist, daß eine Drehung des Elektrodenträgers und damit der Elektrode um die Längsmittelachse des Elektrodenträgers mit der einen Spule erfaßt werden kann.

Wenn der Elektrodenträger, wie dies bei herkömmlichen Instrumenten dieser Art üblich ist, zwei nebeneinander angeordnete Trägerteile aufweist, ist es weiterhin bevorzugt, wenn der zumindest eine Positionssensor an bzw. in dem einen Trägerteil angeordnet ist, während in dem anderen Trägerteil die HF-Zuleitung für die Elektrode angeordnet ist.

Hierbei ist von Vorteil, daß der Positionssensor ausreichend weit von der HF-Zuleitung beabstandet ist, so daß eine Störung der Positionsbestimmung aufgrund des Hochfrequenzstromes möglichst gering gehalten bzw. ausgeschlossen werden kann. Die Positionsbestimmung kann dadurch sehr genau erfolgen. Es kann vorzugsweise zusätzlich vorgesehen sein, die HF-Zuleitung in dem anderen Trägerteil zusätzlich mit einer Abschirmung zu versehen, um Störeinflüsse durch auf die Hochfrequenzstromzuführung zurückgehende elektromagnetische Felder vollständig auszuschließen.

In diesem Zusammenhang ist es bevorzugt, wenn eine mit dem Positionssensor verbundene elektrische Leitung durch das Trägerteil, an dem der Positionssensor angeordnet ist, bis zu einem proximalen Ende dieses Trägerteils verläuft.

In Verbindung mit der zuvor erwähnten bevorzugten Ausgestaltung wird somit bei einem zweiteiligen Elektrodenträger das eine Trägerteil für die HF-Zuleitung genutzt, während das andere Trägerteil vollständig für die Positions- und Lagebestimmung genutzt wird, ohne daß an dem Elektrodenträger sonstige geometrische und bauliche Veränderungen gegenüber einem herkömmlichen Elektrodenträger und einer herkömmlichen Elektrode vorgenommen werden müssen. Ein Elektrodenträger gemäß der vorliegenden Erfindung kann daher auch mit herkömmlichen Instrumentenschäften und Endoskopen kombiniert werden. Über die mit dem Positionssensor verbundene elektrische Leitung wird der Positionssensor je nach Art des verwendeten elektromagnetischen Positions-Erfassungssystems angesteuert und/oder werden die von dem Positionssensor erzeugten Signale nach extrakorporal geführt, bspw. zu einer Auswertevorrichtung.

In einer weiteren bevorzugten Ausgestaltung ist das proximale Ende des Trägerteils, an bzw. in dem der zumindest eine Positionssensor angeordnet ist, als Steckkontakt ausgebildet, so daß er durch axiales Einstecken mit einer Steckerbuchse an einem Instrumentengehäuseteil verbindbar ist.

Hierbei ist von Vorteil, daß die Handhabung des Instruments sich hinsichtlich des Zusammenbaus bzw. Einbaus des Elektrodenträgers mit der Elektrode in ein Instrumentengehäuse bzw. Instrumentenschaft nicht von einem solchen herkömmlichen Instrument unterscheidet, das keine Positionserfassung für die zumindest eine Elektrode aufweist.

In einer weiteren bevorzugten Ausgestaltung ist zumindest ein zweiter elektromagnetisch wirkender Positionssensor an dem Elektrodenträger vorgesehen.

Hierbei ist von Vorteil, daß bspw. im Zusammenhang mit der Ausgestaltung des zumindest einen Positionssensors als Spule, dessen Spulenachse im wesentlichen parallel zur Längsrichtung des Elektrodenträgers angeordnet ist, mittels des zweiten Positionssensors auch der noch invariante Freiheitsgrad einer Drehlage der Elektrode um die Spulenachse erfaßt werden kann, wodurch mit nur zwei Positionssensoren eine vollständige Positions- und Lageerfassung der zumindest einen Elektrode ermöglicht wird. Dieser zusätzliche Positionssensor kann ebenfalls im distalen Bereich des Elektrodenträgers bspw. am gegenüberliegenden Trägerteil oder vorzugsweise in einem proximalen Bereich des Elektrodenträgers angeordnet sein, oder sogar an einem extrakorporalen Teil des Instruments. Dieser zusätzliche Positionssensor kann im letzteren Fall bspw. auch ein optischer Sensor sein.

Dabei ist es weiterhin bevorzugt, wenn der zweite Positionssensor eine im wesentlichen mit ihrer Spulenachse quer zur Längsachse des Elektrodenträgers angeordnete Spule ist.

Das erfindungsgemäße medizinische System umfaßt ein Instrument nach einer oder mehreren der vorhergehenden Ausgestaltungen.

In einer bevorzugten Ausgestaltung des Systems umfaßt dieses einen extrakorporalen Magnetfeldgenerator, der mittels tetraedrisch angeordneter Spulen ein inhomogenes Magnetfeld erzeugt, über das der zumindest eine Positionssensor anregbar ist.

Ein derartiger Magnetfeldgenerator wird von der Firma Mednetix AG, Schweiz, unter dem Handelsnamen AURORA vertrieben und eignet sich aufgrund seiner kompakten Ausgestaltung insbesondere für die Anwendung in der Chirurgie.

In einer weiteren bevorzugten Ausgestaltung des Systems umfaßt dieses eine Auswertevorrichtung, die die von dem zumindest einen Positionssensor erzeugten Positionssignale hinsichtlich der Position und/oder Lage der Elektrode in Korrelation zu einer räumlichen Referenz auswertet.

Die Auswertevorrichtung ermöglicht es, die von dem Positionssensor erzeugten Signale in eine Positionsinformation zu verarbeiten, die dann vorteilhafterweise zur Navigation des Instruments bei einem chirurgischen Einsatz genutzt werden kann.

Dabei ist es weiterhin bevorzugt, wenn ein bildgebendes System vorhanden ist, und wenn die von der Auswertevorrichtung ausgewertete Positionsinformation an das bildgebende System zwecks bildlicher Darstellung der Position und/oder Lage der Elektrode relativ zu der räumlichen Referenz übertragen wird.

Mit dieser Ausgestaltung kann der das Instrument bedienende Arzt die Position und/oder Lage der Elektrode im oder am Gewebe bspw. bei der Durchführung eines Schnittes oder einer Koagulation oder einer Vaporisation kontrollieren, wenn als räumliche Referenz bspw. das Operationsgebiet selbst dargestellt wird.

Wenn das System wie üblich weiterhin mit einem HF-Generator zur Erzeugung des Hochfrequenzstroms ausgestattet ist, mit dem die zumindest eine Elektrode beaufschagt wird, ist es in einer weiteren bevorzugten Ausgestaltung vorgesehen, daß eine Ansteuerungsvorrichtung für die Hochfrequenzstromzufuhr und den Magnetfeldgenerator und/oder die Auswertevorrichtung und/oder den Positionssensor vorgesehen ist, die die Hochfrequenzstromzufuhr deaktiviert, wenn der Magnetfeldgenerator bzw. die Auswertevorrichtung bzw. der Positionssensor aktiviert ist, und umgekehrt.

Diese Maßnahme hat den Vorteil, daß ausgeschlossen werden kann, daß der Hochfrequenzstrom, mit dem das Gewebe behandelt werden soll, die Positionsbestimmung bzw. Lagebestimmung der zumindest einen Elektrode stört bzw. verfälscht. Mit anderen Worten wird eine Positionsbestimmung nur durchgeführt, wenn kein therapeutischer Hochfrequenzstrom fließt, und umgekehrt.

In einer weiteren bevorzugten Ausgestaltung ist eine Verriegelungsschaltung vorgesehen, die wechselweise die Hochfrequenzstromzuführung zu der Elektrode und die Positionserfassung mittels des Positionssensors über zwei direkte elektrische Elektrodenzuleitungen ermöglicht.

Hierbei ist von Vorteil, daß die Hochfrequenz-Stromapplikation und die Positions- bzw. Lagebestimmung der zumindest einen Elektrode über nur zwei, gemeinsam genutzte, direkte elektrische Zuleitungen betrieben werden können, von denen vorzugsweise jeweils eine in einem der beiden nebeneinander angeordneten Trägerteile des Elektrodenträgers angeordnet sein kann. Hierdurch wird ein einfacher und bis auf das Vorhandensein des Positionssensors in einem der beiden Trägerteile nahezu symmetrischer Aufbau des Elektrodenträgers nebst Elektrode erreicht, und es sind ferner nur zwei Steckkontakte für die vorstehend genannten Funktionen notwendig.

Weitere Vorteile und Merkmale ergeben sich aus der nachfolgenden Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel ist in der Zeichnung dargestellt und wird in bezug auf diese hiernach näher beschrieben. Es zeigen:
- Fig. 1: ein medizinisches Instrument zur Behandlung von Gewebe mittels Hochfrequenzstrom in Seitenansicht zusammen mit einer schematischen Darstellung eines medizinischen Systems, von dem das Instrument in Fig. 1 einen Bestandteil bildet;
- Fig. 2: einen Längsschnitt durch einen distalseitigen Abschnitt des Instruments in Fig. 1 in vergrößertem Maßstab, wobei in Fig. 2 eine Elektrode sowie ein E-lektrodenträger des Instruments in Seitenansicht dargestellt sind;
- Fig. 3: eine Draufsicht auf den gesamten Elektrodenträger und die Elektrode in Fig. 2 in Alleinstellung;
- Fig. 4: eine Ansicht von vorne auf die Elektrode in Figuren 2 und 3;
- Fig. 5: eine schematische Darstellung des Ausschnitts A in Fig. 3 in noch weiter vergrößertem Maßstab; und
- Fig. 6: ein Schaltbild einer Verriegelungsschaltung zur wechselweisen Hochfrequenzstromzuführung zur Elektrode und Positionsbestimmung der Elektrode.

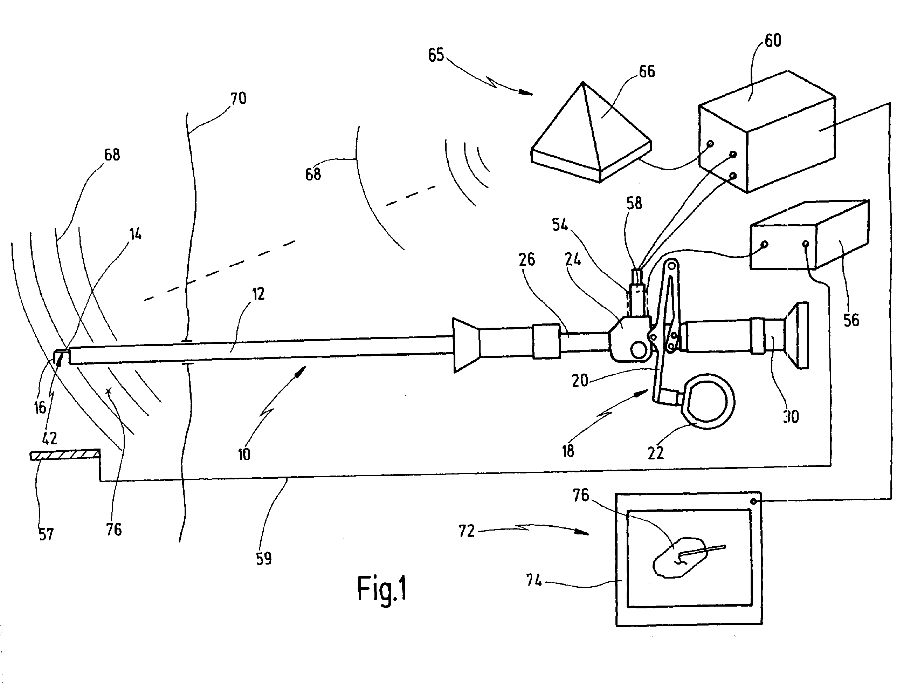

In Fig. 1 ist ein mit dem allgemeinen Bezugszeichen 10 versehenes medizinisches Instrument zur Behandlung von Gewebe mittels Hochfrequenzstrom dargestellt. Das Instrument 10 wird auch als Resektoskop bezeichnet.

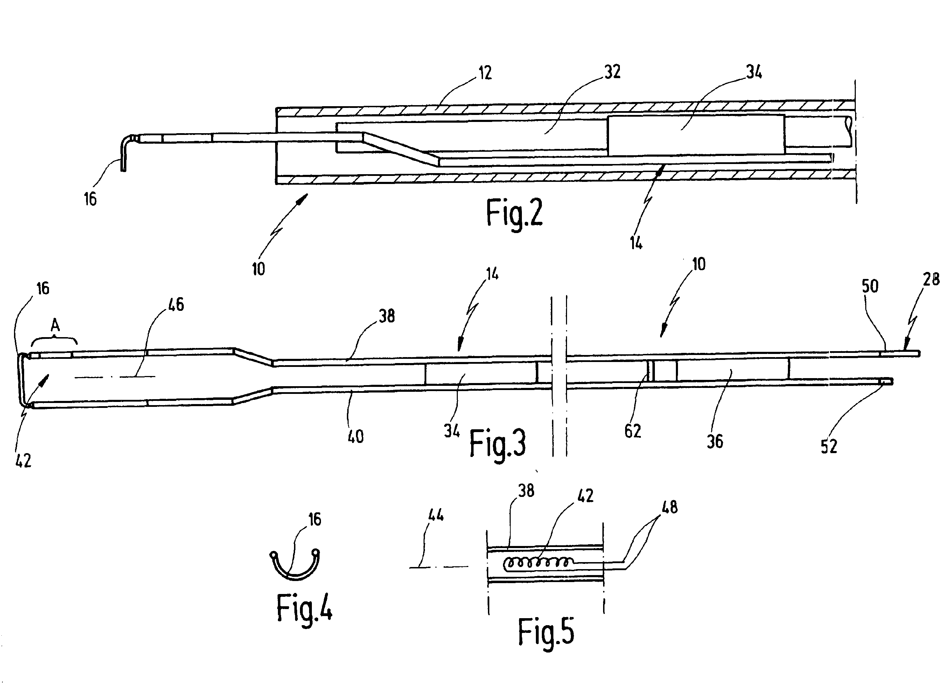

Das Instrument 10 weist einen langerstreckten Schaft 12 auf, in dem gemäß Fig. 2 ein Elektrodenträger 14 angeordnet ist, an dessen distalem Ende eine mit Hochfrequenzstrom beaufschlagbare Elektrode 16 angeordnet ist. Wie aus Fig. 4 hervorgeht, ist die Elektrode 16 als Schlinge ausgebildet. Mit der Elektrode 16 kann unter der Wirkung von Hochfrequenzstrom Gewebe geschnitten, koaguliert und/oder vaporisiert werden.

Der Elektrodenträger 14 und die Elektrode 16 sind relativ zu dem Schaft 12 verschiebbar, wobei in Fig. 2 der Elektrodenträger 14 und die Elektrode 16 in einer aus dem Schaft 12 herausgeschobenen Position dargestellt sind. Die Elektrode 16 kann vollständig in den Schaft 12 zurückgezogen werden.

Um den Elektrodenträger 14 und die Elektrode 16 axial relativ zum Schaft verschieben zu können, ist am proximalen Ende des Instruments 10 eine Handhabe 18 vorgesehen. Die Handhabe 18 weist einen beweglichen Arm 20 auf, an dem ein Bedienungselement 22 befestigt ist, und der an einem Gehäuseteil 24 angelenkt ist. Das Gehäuseteil 24 ist seinerseits mit einer Hülse 26 verbunden, die zusammen mit dem Gehäuseteil 24 relativ zu dem Schaft 12 axial beweglich ist.

Der Elektrodenträger 14 ist mit seinem proximalen Ende 28 (Fig. 3) mit dem Gehäuseteil 24 verbunden, wie hiernach noch erläutert wird.

Durch den Schaft 12 des Instruments 10 erstreckt sich ferner eine Endoskopoptik, die gemäß Fig. 1 ein Okular 30 sowie einen mit diesem verbundenen Endoskopschaft 32 gemäß Fig. 2 aufweist. Der Endoskopschaft 32 ist relativ zu dem Schaft 12 unbeweglich, während der Elektrodenträger 14 mittels Klemmen 34 und 36 (Figuren 2 und 3) an dem Endoskopschaft 32 gleitend geführt ist.

Der Elektrodenträger 14 weist ein erstes Trägerteil 38 und ein zweites Trägerteil 40 auf, die beide nebeneinander angeordnet sind und im wesentlichen parallel zueinander verlaufen. Über die Klemmen 34 und 36 erfahren die beiden Trägerteile 38 und 40 eine Fixierung aneinander. Dennoch ist der Elektrodenträger 14 relativ labil und kann sich beim Anwenden von Druck über die Elektrode 16 auf zu behandelndes Gewebe durchbiegen.

Soweit unterscheidet sich der Elektrodenträger 14 nebst der Elektrode 16 im wesentlichen nicht von einem herkömmlichen Elektrodenträger und einer herkömmlichen Elektrode.

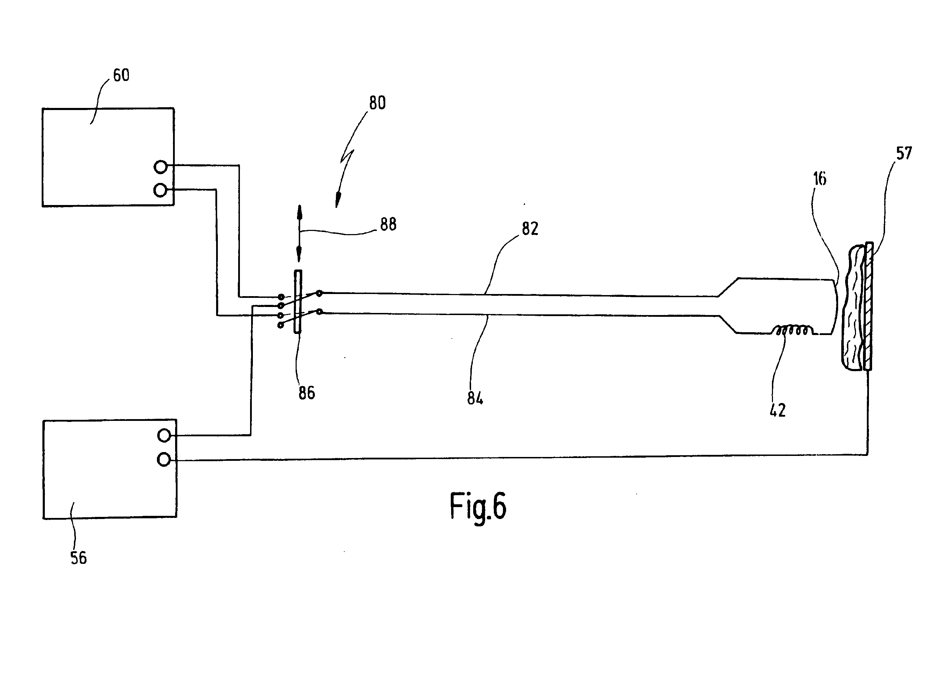

Ein wesentlicher Unterschied zwischen einem herkömmlichen Elektrodenträger und dem Elektrodenträger 14 besteht darin, daß in einem in Fig. 3 mit A bezeichneten distalen Bereich des Elektrodenträgers 14 der Elektrode 16 unmittelbar benachbart ein elektromagnetisch wirkender Positionssensor 42 angeordnet ist, der gemäß Fig. 5 als Spule ausgebildet ist. Der Positionssensor 42 ermöglicht es, beim Gebrauch des Instruments 10, bspw. bei einer Prostataresektion, die intrakorporale Position und die Lage der Elektrode 16 und damit auch die Eindringtiefe der Elektrode 16 in das zu behandelnde Gewebe zu erfassen, wie hiernach noch beschrieben wird. '

Der Positionssensor 42 in Form der Spule ist dabei in den Körper des Elektrodenträgers 14, genauer gesagt, in den Körper des ersten Trägerteils 38 nahe der Elektrode 16 eingebettet, wie in Fig. 5 schematisch veranschaulicht ist. Im Bereich der Spule ist das Trägerteil 38 aus einem Material, das gegenüber elektromagnetischen Feldern nicht abschirmend bzw. nicht verzerrend wirkt.

Der Positionssensor 42 in Form der Spule ist dabei mit der Spulenachse 44 parallel zur Längsrichtung des Elektrodenträgers 14 angeordnet.

Die den Positionssensor 42 bildende Spule weist einen Durchmesser von weniger als 2 mm, vorzugsweise weniger als 1 mm auf, und besitzt eine Länge im Bereich von etwa 5-10 mm.

Mit der den Positionssensor 42 bildenden Spule ist es möglich, die räumliche Position und Lage der Elektrode 16 bezüglich einer räumlichen Referenz in den drei kartesischen Raumrichtungen x, y und z zu bestimmen, sowie die Drehlage der Elektrode 16 um zwei zur Spulenachse 44 senkrechte, senkrecht zueinander stehende Achsen.

Wie aus Fig. 3 hervorgeht, ist der Positionssensor 42 ferner außerhalb der Längsmittelachse 46 des Elektrodenträgers 14 angeordnet, so daß eine Drehung der Elektrode 16 um die Längsmittelachse 46 des Elektrodenträgers 14 mit dem Positionssensor 42 erfaßt werden kann. Nicht erfaßt werden kann mit dem Positionssensor 42 eine Drehung der Elektrode 16 um die Spulenachse 44 der den Positionssensor 42 bildenden Spule.

Während der Positionssensor 42 in dem ersten Trägerteil 38 des Elektrodenträgers 14 angeordnet ist, verläuft die HF-Zuleitung (nicht dargestellt) für die Elektrode 16 ausschließlich in dem zweiten Trägerteil 40, und zwar von der Elektrode 16 bis zu dem proximalen Ende 28 des Elektrodenträgers 14.

In dem ersten Trägerteil 38 verläuft dagegen eine mit dem Positionssensor 42 verbundene elektrische Leitung 48, und zwar von dem Positionssensor 42 bis zu dem proximalen Ende 28 des Elektrodenträgers 14. Die elektrische Leitung 48 dient als Signalleitung für die von dem Positionssensor 42 infolge elektromagnetischer Anregung erzeugten Signale durch das erste Trägerteil 38 hindurch nach proximal und extrakorporal. Anstelle einer Signalübertragung mittels einer elektrischen Leitung kann jedoch auch eine telemetrische bzw. drahtlose Signalübertragung nach extrakorporal vorgesehen sein.

Wie bei einem herkömmlichen Elektrodenträger weist das erste Trägerteil 38 am proximalen Ende 28 des Elektrodenträgers 14 einen Steckkontakt 50 und das zweite Trägerteil 40 einen Steckkontakt 52 auf, über die der Elektrodenträger 14 in dem Gehäuseteil 24 des Instruments 10 verrastet wird. Im Unterschied zu einem herkömmlichen Elektrodenträger dient jedoch lediglich der Steckkontakt 52 des Elektrodenträgers 14 dazu, über einen HF-Steckeranschluß 54 (Fig. 1) mit einem HF-Generator 56 verbunden zu werden, um die Elektrode 16 mit Hochfrequenzstrom entsprechend zu beaufschlagen. Eine Neutralelektrode 57, die üblicherweise extrakorporal am Körper des Patienten anliegt, ist ebenfalls mit dem HF-Generator 56 über eine elektrische Leitung 59 verbunden.

Der Steckkontakt 50 des ersten Trägerteils 38 wird ebenfalls wie herkömmlich in eine entsprechende Steckerbuchse im Gehäuseteil 24 (nicht dargestellt) eingesteckt, steht jedoch über diese mit einem neben dem HF-Steckeranschluß 54 angeordneten weiteren Steckeranschluß 58 in Verbindung, über den das Instrument 10 mit einer Ansteuerungs-/Auswertevorrichtung 60 verbunden wird, die für die Positions- und Lagebestimmung der Elektrode 16 dient.

Gemäß Fig. 3 ist an dem Elektrodenträger 14 ein zweiter elektromagnetisch wirkender Positionssensor 62 an einer weiter proximalen Stelle des Elektrodenträgers 14 angeordnet, der ebenfalls als Spule ausgebildet sein kann und mit seiner Spulenachse im wesentlichen quer zur Längsrichtung des Elektrodenträgers 14 angeordnet ist. Mittels dieses zweiten Positionssensors 62 kann dann die Invarianz der Positionsbestimmung gegenüber einer Drehung der Elektrode 16 um die Spulenachse 44 der den ersten Positionssensor 42 bildenden Spule aufgehoben werden, so daß dann Position und Lage der Elektrode 16 im Raum eindeutig bestimmt werden kann, wodurch das Eindringen der Elektrode 16 in das zu behandelnde Gewebe sehr gut kontrolliert werden kann.

In Fig. 1 ist neben dem medizinischen Instrument 10 schematisch ein medizinisches System 65 dargestellt, zu dem das Instrument 10 gehört.

Neben dem Instrument 10 umfaßt das System 65 den bereits genannten HF-Generator 56 sowie die Ansteuerungs-/Auswertevorrichtung 60.

Zusätzlich weist das System 65 einen Magnetfeldgenerator 66 auf, der ein elektromagnetisches Feld 68 extrakorporal erzeugt, wobei das elektromagnetische Feld 68 durch die Körperoberfläche 70 in den Körper eingekoppelt wird.

Der Magnetfeldgenerator 66 weist tetraedrisch angeordnete, gepulst angeregte Spulen auf, mittels der das elektromagnetische Feld 68 als inhomogenes Feld erzeugt wird, wobei der Positionssensor 42 durch das Feld 68 angeregt wird. Je nach Orientierung des Positionssensors 42 relativ zu dem inhomogenen elektromagnetischen Feld 68 wird ein entsprechendes Signal in dem Positionssensor 42 erzeugt, das dann nach extrakorporal übertragen wird. Der Magnetfeldgenerator 66 erzeugt dabei eine Folge bzw. einen Satz von bspw. sechs unterschiedlichen räumlichen Magnetfeldgeometrien bzw. -verteilungen, von denen jede von dem Positionssensor 42 erfaßt wird. Jede Folge von unterschiedlichen Magnetfeldverteilungen erzeugt eine Signalfolge in dem Positionssensor 42. Die Verarbeitung der Signalfolge in der Ansteuerungs-/Auswertevorrichtung 60 ermöglicht dann die Bestimmung der Lage des Positionssensors 42 und damit die Lage der Elektrode 16 relativ zu einer räumlichen Referenz, die in fester Beziehung zu dem Magnetfeldgenerator 66 steht.

Der Magnetfeldgenerator 66 ist bspw. ein unter der Handelsbezeichnung AURORA der Firma Mednetix, Schweiz, vertriebener Magnetfeldgenerator.

Die von dem Positionssensor 42 aufgrund der Wechselwirkung mit dem extrakorporal eingestrahlten elektromagnetischen Feld 68 erzeugten Signale werden über den Steckeranschluß 58 an die Ansteuerungs-/Auswertevorrichtung 60 übertragen und dort zur Bestimmung der Position und Lage der Elektrode 16 in Korrelation zu einer räumlichen Referenz, bspw. dem Operationsgebiet bzw. dem Körper des Patienten, ausgewertet.

Das System 65 umfaßt ferner ein bildgebendes System 72, von dem in Fig. 1 lediglich die Visualisierungsvorrichtung in Form eines Bildschirmes dargestellt ist. Das bildgebende System 72 kann bspw. ein Ultraschall-, Computertomographie-, optische Kohärenztomographie- oder Magnetresonanzsystem sein, mit dem es möglich ist, Schnittbilder vom Operationsgebiet zu erhalten und auf der Visualisierungsvorrichtung 74 darzustellen. In Fig. 1 ist das so dargestellte Operationsgebiet mit dem Bezugszeichen 76 versehen. Die von der Ansteuerungs-/Auswertevorrichtung ausgewertete Positionsinformation bezüglich der Position und/oder Lage der Elektrode 16 wird zur bildlichen Darstellung der Position und/oder Lage der Elektrode 16 relativ zu dem Operationsgebiet 76 auf das bildgebende System 72 übertragen und auf der Visualisierungsvorrichtung 74 in räumlicher Korrelation zu dem Operationsgebiet 76 bildlich dargestellt. Auf diese Weise kann die Positions- und/oder Lagebestimmung der Elektrode 16 zur Navigation des Instruments 10 im Operationsgebiet 76 benutzt werden.

Der Magnetfeldgenerator 66 ist wie das Instrument 10 mit der Ansteuerungs-/Auswertevorrichtung 60 verbunden. Auch der HF-Generator 56 ist mit der Ansteuerung-/Auswertevorrichtung 60 verbunden. Die Ansteuerungs-/Auswertevorrichtung 60 deaktiviert den HF-Generator 56, wenn der Magnetfeldgenerator 66 aktiviert ist, und umgekehrt. Auf diese Weise erfolgt keine Positions- und/oder Lagebestimmung der Elektrode 16, wenn die Elektrode 16 mit Hochfrequenzstrom beaufschlagt ist, und umgekehrt. Eine Verfälschung bzw. Störung der Positionsbestimmung der Elektrode 16 wird dadurch sicher vermieden.

Hierzu weist bspw. die Ansteuerungsvorrichtung 60 gemäß Fig. 6 eine Verriegelungsschaltung 80 auf, die wechselweise die Hochfrequenzstromzuführung zu der Elektrode 16 und die Positionserfassung mittels des Positionssensors 42 über zwei direkte elektrische Elektrodenzuleitungen 82 und 84 ermöglicht. Die Elektrodenzuleitungen 82 und 84 sind über einen Umschalter 86 wechselweise mit der Ansteuerungs-/Auswertevorrichtung 60 und dem HF-Generator 56 verbunden. In der in Fig. 6 mit durchgezogenen Linien dargestellten Betriebsstellung des Umschalters 86 sind die Zuleitungen 82 und 84 mit dem HF-Generator 56 verbunden, so daß in dieser Stellung des Umschalters 86 die Elektrode 16 mit Hochfrequenzstrom beaufschlagt wird. In der mit unterbrochenen Linien dargestellten Betriebsstellung des Umschalters 86 sind die Zuleitungen 82 und 84 mit der Ansteuerungs- /Auswerteeinheit 60 verbunden, wodurch die Elektrodenzuleitungen 82 und 84 den Positionssensor 42 in Form der Spule zur Positions- bzw. Lagebestimmung der Elektrode 16 mit der Ansteuerungs-/Auswertevorrichtung 60 verbinden. Das wechselweise Umschalten des Umschalters 86 gemäß einem Doppelpfeil 88 kann dabei gemäß einem vorgegebenen Takt automatisch periodisch oder aperiodisch erfolgen oder auch durch eine nicht dargestellte manuelle Betätigungseinrichtung, die vom das Instrument bedienenden Arzt betätigt werden kann.

## Patentansprüche

1. Medizinisches Instrument zur Behandlung von Gewebe mittels Hochfrequenz strom, mit zumindest einer mit Hochfrequenzstrom beaufschlagbaren Elektrode (16), die an einem distalen Ende eines langerstreckten Elektrodenträgers (14) angeordnet ist, **dadurch gekennzeichnet, daß** im distalen Bereich (A) des Elektrodenträgers (14) zumindest ein elektromagnetisch wirkender Positionssensor (42), der in der Lage ist, in einem Magnetfeld ein elektrisches Signal zu erzeugen, der zumindest einen Elektrode (16) unmittelbar benachbart angeordnet ist, derart, daß die intrakorporale Position und/oder die Lage der zumindest einen Elektrode (16) erfaßbar ist.

2. Instrument nach Anspruch 1, **dadurch gekennzeichnet, daß** der zumindest eine Positionssensor (42) im Körper des Elektrodenträgers (14) nahe der Elektrode (16) eingebettet ist.

3. Instrument nach Anspruch 1 oder 2, **dadurch gekennzeichnet, daß** der Positionssensor (42) eine Spule ist.

4. Instrument nach Anspruch 3, **dadurch gekennzeichnet, daß** die Spule mit ihrer Spulenachse (44) im wesentlichen parallel zur Längsrichtung des Elektrodenträgers (14) angeordnet ist.

5. Instrument nach einem der Ansprüche 1 bis 4, **dadurch gekennzeichnet, daß** der zumindest eine Positionssensor (42) außerhalb der Längsmittelachse (46) des Elektrodenträgers (14) angeordnet ist.

6. Instrument nach einem der Ansprüche 1 bis 5, wobei der Elektrodenträger (14) zwei nebeneinander angeordnete Trägerteile (38, 40) aufweist, **dadurch gekennzeichnet, daß** der zumindest eine Positionssensor (42) an bzw. in dem einen Trägerteil (38) angeordnet ist, während in dem anderen Trägerteil (40) die HF-Zuleitung für die Elektrode (16) angeordnet ist.

7. Instrument nach Anspruch 6, **dadurch gekennzeichnet, daß** eine mit dem Positionssensor (42) verbundene elektrische Leitung (48) durch das Trägerteil (38), an dem der Positionssensor (42) angeordnet ist, bis zu einem proximalen Ende dieses Trägerteils (38) verläuft.

8. Instrument nach Anspruch 7, **dadurch gekennzeichnet, daß** das proximale Ende des Trägerteils (38), an bzw. in dem der zumindest eine Positionssensor (42) angeordnet ist, als Steckkontakt (50) ausgebildet ist, so daß er durch axiales Einstecken mit einer Steckerbuchse an einem Instrumentengehäuseteil (24) verbindbar ist.

9. Instrument nach einem der Ansprüche 1 bis 8, **dadurch gekennzeichnet, daß** zumindest ein zweiter elektromagnetisch wirkender Positionssensor (62) an dem Elektrodenträger (14) vorgesehen ist.

10. Instrument nach Anspruch 9, **dadurch gekennzeichnet, daß** der zweite Positionssensor (62) eine im wesentlichen mit ihrer Spulenachse quer zur Längsrichtung des Elektrodenträgers (14) angeordnete Spule ist.

11. Medizinisches System, **gekennzeichnet durch** ein Instrument (10) nach einem der Ansprüche 1, bis 10.

12. System nach Anspruch 11, weiterhin **gekennzeichnet durch** einen extrakorporalen Magnetfeldgenerator (66), der mittels tetraedrisch angeordneter Spulen ein inhomogenes Magnetfeld (68) erzeugt, über das der zumindest eine Posi-tionssensor (42, 62) anregbar ist.

13. System nach Anspruch 11 oder 12, weiterhin **gekennzeichnet durch** eine Auswertevorrichtung (60), die die von dem zumindest einen Positionssensor (42, 62) erzeugten Positionssignale hinsichtlich der Position und/oder Lage der Elektrode (16) in Korrelation zu einer räumlichen Referenz (76) auswertet.

14. System nach Anspruch 13, **dadurch gekennzeichnet, daß** ein bildgebendes System (72) vorhanden ist, und daß die von der Auswertevorrichtung (60) ausgewertete Positionsinformation an das bildgebende System (72) zwecks, bildlicher Darstellung der Position und/oder Lage der Elektrode (16) relativ zu der räumlichen Referenz (76) übertragen wird.

15. System nach einem der Ansprüche 11 bis 14, weiterhin mit einem HF-Generator (56) zur Erzeugung des Hochfrequenzstroms, **gekennzeichnet durch** eine Ansteuerungsvorrichtung (60) für die Hochfrequenzstromzufuhr und den Magnetfeldgenerator (66) und/oder die Auswertevorrichtung (60) und/oder den Positionssensor (42, 62), die die Hochfrequenzstromzufuhr deaktiviert, wenn der Magnetfeldgenerator (66) bzw. die Auswertevorrichtung (60) bzw. der Positionssensor (42, 62) aktiviert ist, und umgekehrt.

16. System nach Anspruch 15, **dadurch gekennzeichnet, daß** eine Verriegelungsschaltung (80) vorgesehen ist, die wechselweise die Hochfrequenzstromzuführung zu der Elektrode (16) und die Positionserfassung mittels des Positionssensors. (42, 62) über zwei direkte elektrische Elektrodenzuleitungen (82, 84) ermöglicht.

## Claims

1. A medical instrument for the treatment of tissue by means of high-frequency current, comprising at least one electrode (16) which can be supplied with high-frequency current and is arranged at a distal end of an elongate electrode carrier (14), **characterized in that** at least one electromagnetically acting position sensor (42) in the distal region (A) of the electrode carrier (14) is arranged immediately adjacent to the at least one electrode (16) in such a way that the intracorporeal position and/or the orientation of the at least one electrode (16) can be sensed.

2. The instrument of claim 1, **characterized in that** the at least one position sensor (42) is embedded in the body of the electrode carrier (14) near the electrode (16).

3. The instrument of claim 1 or 2, **characterized in that** the position sensor (42) is a coil.

4. The instrument of claim 3, **characterized in that** the coil is arranged with its coil axis (44) substantially parallel to the longitudinal direction of the electrode carrier (14).

5. The instrument of any one of claims 1 through 4, **characterized in that** the at least one position sensor (42) is arranged outside the longitudinal center axis (46) of the electrode carrier (14).

6. The instrument of any one of claims 1 through 5, the electrode carrier (14) having two carrier parts (38, 40) arranged next to each other, **characterized in that** the at least one position sensor (42) is arranged on or in the one carrier part (38), while the HF supply line for the electrode (16) is arranged in the other carrier part (40).

7. The instrument of claim 6, **characterized in that** an electrical line (48) connected to the position sensor (42) runs through the carrier part (38) on which the position sensor (42) is arranged up to a proximal end of this carrier part (38).

8. The instrument of claim 7, **characterized in that** the proximal end of the carrier part (38) on or in which the at least one position sensor (42) is arranged is formed as a plug-in contact (50), so that it can be connected to a socket on an instrument housing part (24) by axial insertion.

9. The instrument of any one of claims 1 through 8, **characterized in that** at least a second electromagnetically acting position sensor (62) is provided on the electrode carrier (14).

10. The instrument of claim 9, **characterized in that** the second position sensor (62) is a coil arranged substantially with its coil axis transversely in relation to the longitudinal axis of the electrode carrier (14).

11. A medical system, **characterized by** an instrument (10) of any one of claims 1 through 10.

12. The system of claim 11, further **characterized by** an extracorporeal magnetic-field generator (66), which generates by means of tetrahedrally arranged coils an inhomogeneous magnetic field (68), by means of which the at least one position sensor (42, 62) can be excited.

13. The system of claim 11 or 12, further **characterized by** an evaluating device (60), which evaluates the position signals generated by the at least one position sensor (42, 62) with regard to the position and/or orientation of the electrode (16) in correlation with a spatial reference (76).

14. The system of claim 13, **characterized in that** an image generating system (72) is present, and **in that** the positional information evaluated by the evaluating device (60) is transmitted to the image generating system (72) for the purpose of graphic representation of the position and/or orientation of the electrode (16) in relation to the spatial reference (76).

15. The system of any one of claims 11 through 14, further comprising an HF generator (56) for generating the high-frequency current, **characterized by** a driving device (60) for the high-frequency current supply and the magnetic-field generator (66) and/or the evaluating device (60) and/or the position sensor (42, 62), which device deactivates the high-frequency current supply when the magnetic-field generator (66) or the evaluating device (60) or the position sensor (42, 62) is activated, and vice versa.

16. The system of claim 15, **characterized in that** an interlocking circuit (80) which alternately makes possible the high-frequency current supply to the electrode (16) and the position sensing by means of the position sensor (42, 62) via two direct electrical electrode supply lines (82, 84) is provided.

## Revendications

1. Instrument médical pour le traitement de tissu à l'aide d'un courant à haute fréquence, avec au moins une électrode (16) alimentée en courant de haute fréquence qui est disposée à une extrémité distale d'un support d'électrode allongé (14), **caractérisé en ce qu'**au moins un détecteur de position (42) à action électromagnétique qui est capable de générer un signal électrique dans un champ magnétique est disposé dans la zone distale (A) du support d'électrode (14) directement à côté de la au moins une électrode (16) de manière à ce que la position intracorporelle et/ou l'état intracorporel de la au moins une électrode (16) soit détectable.

2. Instrument selon la revendication 1, **caractérisé en ce que** le au moins un détecteur de position (42) est encastré dans le corps du support d'électrode (14) à proximité de l'électrode (16).

3. Instrument selon la revendication 1 ou 2, **caractérisé en ce que** le détecteur de position (42) est une bobine.

4. Instrument selon la revendication 3, **caractérisé en ce que** la bobine est disposée avec son axe de bobine (44) sensiblement parallèlement à la direction longitudinale du support d'électrode (14).

5. Instrument selon l'une quelconque des revendications 1 à 4, **caractérisé en ce que** le au moins un détecteur de position (42) est disposé à l'extérieur de l'axe médian longitudinal (46) du support d'électrode (14).

6. Instrument selon l'une quelconque des revendications 1 à 5, dans lequel le support d'électrode (14) comprend deux parties de support (38, 40) disposées l'une à côté de l'autre, **caractérisé en ce que** le au moins un détecteur de position (42) est disposé sur ou dans l'une des parties de support (38) alors que le câble d'alimentation HF pour l'électrode (16) est disposé dans l'autre partie de support (40).

7. Instrument selon la revendication 6, **caractérisé en ce qu'**un câble électrique (48) relié au détecteur de position (42) s'étend à travers la partie de support (38) sur laquelle est disposé le détecteur de position (42) jusqu'à une extrémité proximale de cette partie de support (38).

8. Instrument selon la revendication 7, **caractérisé en ce que** l'extrémité proximale de la partie de support (38) est disposée sur ou dans le au moins un détecteur de position (42), **en ce qu'**elle est configurée comme un contact à fiche (50) de sorte qu'elle peut être reliée par enfichage axial à une prise femelle dans une partie de boîtier d'instrument (24).

9. Instrument selon l'une quelconque des revendications 1 à 8, **caractérisé en ce qu'**il est prévu au moins un deuxième détecteur de position (62) à action électromagnétique sur le support d'électrode (14).

10. Instrument selon la revendication 9, **caractérisé en ce que** le deuxième détecteur de position (62) est une bobine disposée avec son axe de bobine sensiblement perpendiculairement à la direction longitudinale du support d'électrode (14).

11. Système médical **caractérisé par** un instrument (10) selon l'une quelconque des revendications 1 à 10.

12. Système selon la revendication 11, **caractérisé, en outre, par** un générateur de champ magnétique extracorporel (66) qui génère un champ magnétique inhomogène (68) à l'aide de bobines disposées en forme de tétraèdre, au moyen duquel le au moins un détecteur de position (42, 62) est excitable.

13. Système selon la revendication 11 ou 12, **caractérisé, en outre, par** un dispositif d'évaluation (60) qui évalue les signaux de position générés par le au moins un détecteur de position (42, 62) concernant la position et/ou l'état de l'électrode (16) en corrélation avec une référence spatiale (76).

14. Système selon la revendication 13, **caractérisé en ce qu'**il est prévu un système d'imagerie (72) et **en ce que** l'information de position évaluée par le dispositif d'évaluation (60) est transmise au système d'imagerie (72) en vue d'une représentation par images de la position et/ou de l'état de l'électrode (16) par rapport à la référence spatiale (76).

15. Système selon l'une quelconque des revendications 11 à 14 avec, en outre, un générateur HF (56) pour générer le courant de haute fréquence, **caractérisé par** un dispositif de commande (60) pour l'alimentation de courant de haute fréquence et le générateur de champ magnétique (66) et/ou le dispositif d'évaluation (60) et/ou le détecteur de position (42, 62) qui désactive l'alimentation de courant de haute fréquence lorsque le générateur de champ magnétique (66) ou le dispositif d'évaluation (60) ou le détecteur de position (42, 62) est activé et vice versa.

16. Système selon la revendication 15, **caractérisé en ce qu'**il est prévu un circuit de verrouillage (80) qui permet alternativement l'alimentation de courant de haute fréquence à l'électrode (16) et la détection de position à l'aide du détecteur de position (42, 62) par l'intermédiaire de deux câbles d'alimentation électrique directe (82, 84).
